# EUROPÄISCHE PATENTSCHRIFT

(11) **EP 1 753 408 B1**
(45) Veröffentlichungstag und Bekanntmachung des Hinweises auf die Patenterteilung: **02.01.2019**
(21) Anmeldenummer: 05750711.3
(22) Anmeldetag: 27.05.2005
(51) Int. Cl.: A61K 9/36, A61K 31/565, A61K 31/57

(54) **HORMONALES KONTRAZEPTIVUM ENTHALTEND EINE KOMBINATION AUS ETHINYLESTRADIOL UND CHLORMADINONACETAT**
HORMONAL CONTRACEPTIVE CONTAINING A COMBINATION OF ETHINYL ESTRADIOL AND CHLORMADINONE ACETATE
CONTRACEPTIF HORMONAL CONTENANT UNE ASSOCIATION D'ETHINYLESTRADIOL ET D'ACETATE DE CHLORMADINONE

(30) Priorität: 28.05.2004 DE 102004026670
(43) Veröffentlichungstag der Anmeldung: 21.02.2007
(73) Patentinhaber: Richter Gedeon Nyrt., 1103 Budapest (HU)
(72) Erfinder: KLOSE, Janine, 52146 Würselen (DE); BARTHOLOMÄUS, Johannes, 52080 Aachen (DE); WILSMANN, Klaus-Michael, 52159 Roetgen (DE); SCHRAMM, Georg, 52224 Stolberg (DE)
(74) Vertreter: Kutzenberger Wolff & Partner
(86) Internationale Anmeldenummer: PCT/EP2005/005763
(87) Internationale Veröffentlichungsnummer: WO 2005/115351

(56) Entgegenhaltungen:
- EP-B1- 0 735 883
- DE-A1- 4 321 957
- DE-A1- 4 339 934
- DE-A1- 19 539 233
- US-B1- 6 451 779
- ROTE LISTE SERVICE: "Rote Liste 2002" 2002, EDITIO CANTOR VERLAG , AULENDORF , XP002339882 Absatz [76021]
- RAUDRANT D ET AL: "PROGESTOGENS WITH ANTIANDROGENIC PROPERTIES" DRUGS, ADIS INTERNATIONAL LTD, AT, Bd. 63, Nr. 5, 2003, Seiten 463-492, XP008038646 ISSN: 0012-6667
- SCHRAMM G ET AL: "CONTRACEPTIVE EFFICACY AND TOLERABILITY OF CHLORMADINONE ACETATE 2MG/ETHINYLESTRADIOL 0.03MG (BELARA) RESULTS OF A POST-MARKETING SURVEILLANCE STUDY", CLINICAL DRUG INVESTIGATION, ADIS INTERNATIONAL, AUCKLAND, NZ, vol. 22, no. 4, 1 January 2002 (2002-01-01), pages 221-231, XP009055106, ISSN: 1173-2563, DOI: 10.2165/00044011-200222040-00002
- KUHL H: "AKTUELLE ENTWICK-LUNGEN IN DER HORMONALEN KONTRAZEPTION", GYNAEKOLOGE, SPRINGER VERLAG, BERLIN, vol. 25, no. 4, 1 August 1992 (1992-08-01) , pages 231-240, XP002046681, ISSN: 0017-5994

## Beschreibung

Die vorliegende Erfindung betrifft ein hormonales Kontrazeptivum aus 21 bis 25 hormonhaltigen Tages-Einheiten zur Verabreichung einer Kombination aus einem Östrogen und einem Gestagen pro Tages-Einheit und 7 bis 3 hormonfreien Tages-Einheiten an Frauen, dadurch gekennzeichnet, dass die hormonhaltigen Tages-Einheiten eine Kombination aus Ethinylestradiol in einer Menge von 10 bis 20 µg und Chlormadinonacetat in einer Menge von 1 bis 4 mg enthalten.

Bei Kontrazeptiva wird üblicherweise pro Tages-Einheit eine Kombination aus einem Östrogen und einem Gestagen verabreicht, wobei die Menge an Östrogen möglichst gering gehalten werden soll.

Für alle Frauen, insbesondere aber für Frauen über 35 Jahre und insbesondere für Frauen in der Prä- und Perimenopause ist ein hormonales Kontrazeptivum mit einer möglichst geringeren Menge Östrogen wünschenswert, da Frauen ab dem 35. Lebensjahr ein erhöhtes Risiko haben, durch östrogenhaltige Verhütungsmittel Komplikationen, wie Herzinfarkt, Schlaganfall und Beinvenenthrombosen, gefolgt von Lungenembolien, zu erleiden.

Aus dem Stand der Technik ist bereits bekannt, eine Kombination aus 30 µg Ethinylestradiol und 2 mg Chlormadinonacetat zur Kontrazeption und gegebenenfalls zur gleichzeitigen Behandlung von Akne zu verwenden. Es besteht darüber hinaus der Bedarf, die Menge an Ethinylestradiol, die mit Chlormadinonacetat kombiniert ist, noch weiter zu reduzieren, vorausgesetzt, dass eine zuverlässige kontrazeptive Wirkung trotzdem gewährleistet bleibt.

Weiterhin ist die kontrazeptive Wirkung von Kombinationspräparaten aus 15 µg Ethinylestradiol und 60 µg Gestoden, die während 24 Tagen pro Zyklus verabreicht werden, bereits bekannt (Sullivan et al, Fertility and Sterility 72 (1999)115-120; Gestodene Study Group 322, The European Journal of Contraception and Reproductive Health Care 4 (Suppl. 2) (1999) 9-15). Gestoden gewährleistet aber nicht die vorteilhaften Eigenschaften des Chlormadinonacetats.

Weitere Kombinationspräparate zur hormonalen Kontrazeption und zur gleichzeitigen Behandlung von Beschwerden in der Prä- und Perimenopause sind aus EP 0 398 460 und EP 0 253 607 bekannt, die allerdings kein Chlormadinonacetat als Gestagen enthalten.

Daher war es die Aufgabe der vorliegenden Erfindung, ein hormonales Kontrazeptivum zur Verfügung zu stellen, das Chlormadinonacetat als Gestagen kombiniert mit einer gegenüber dem Stand der Technik geringeren Menge an Ethinylestradiol enthält und trotzdem eine zuverlässige kontrazeptive Wirkung gewährleistet.

Diese Aufgabe wird durch das erfindungsgemäße hormonale Kontrazeptivum, das aus 21 bis 25, vorzugsweise 21 bis 24, hormonhaltigen Tages-Einheiten, die Ethinylestradiol in Mengen von 10 bis 20 µg und Chlormadinonacetat in Mengen von 1 bis 4 mg enthalten und aus 7 bis 3, vorzugsweise 7 bis 4 hormonfreien Tages-Einheiten zur oralen Verabreichung an Frauen besteht, gelöst.

Der Einsatz von Chlormadinonacetat gewährleistet eine hohe kontrazeptive Sicherheit und zeichnet sich durch seine ausgeprägten antiandrogenen Wirkungen aus. Daher ist dieser Einsatz insbesondere für alle Frauen in fertilem Alter geeignet, da viele Frauen an androgenabhängigen Symptomen, wie Akne, Hirsutismus (z. B. Damenbart), androgenetischer Alopezie, Seborrhoe leiden. Darüber hinaus kann bei Frauen mit Regelbeschwerden (Dysmenorrhöe) mit dem erfindungsgemäßen Kontrazeptikum Abhilfe geschaffen werden. Das erfindungsgemäße Kontrazeptivum eignet sich insbesondere auch für die Kontrazeption von Frauen über 35 Jahre, da es das Thromboserisiko vermindert.

Das erfindungsgemäße, hormonale Kontrazeptivum umfasst eine Kombination von Chlormadinonacetat in einer Menge von 1 bis 4 mg, bevorzugt von 1 bis 3 mg, und von Ethinylestradiol in einer Menge von 10 bis 20 µg, ganz besonders bevorzugt von 15 bis 20 µg.

In einer ganz besonders bevorzugten Ausführungsform enthalten alle hormonhaltigen Tages-Einheiten des erfindungsgemäßen Kontrazeptivums jeweils 2 mg Chlormadinonacetat und 20 µg Ethinylestradiol und werden als einphasiges Kontrazeptivum während eines weiblichen Zyklus an 21 bis 24 aufeinanderfolgenden Tagen, gefolgt von der Einnahme von hormonfreien Tages-Einheiten über 7 bis 4 Tage eingenommen.

Das erfindungsgemäße Kontrazeptivum kann auch als mehrphasiges Kontrazeptivum aufgebaut sein. Bei einem mehrphasigen Kontrazeptivum kann eine Zweiphasen- oder eine Dreiphasen-Pillen-Ennahme vorgesehen sein.

Die hormonhaltigen Tages-Einheiten eines mehrphasigen erfindungsgemäßen Kontrazeptivums weisen vorzugsweise eine Menge von gleich oder weniger 20 µg Ethinylestradiol (EE), vorzugsweise 20 µg Ethinylestradiol über alle Phasen und eine phasenabhängig, unterschiedliche Menge von 1 bis 3 mg Chlormadinonacetat auf.

Bei einem Zweiphasen-Kontrazeptivum beginnt der Zyklus vorzugsweise mit einer täglichen Einnahme einer Tages-Einheit enthaltend 1 bis 2 mg Chlormadinonacetat neben EE über einen Zeitraum von 7 bis12 Tagen und endet mit einer täglichen Einnahme einer Tages-Einheit von 2 bis 3 mg Chlormadinonacetat über einen Zeitraum von 9 bis 18 Tagen, wobei die Menge des Chlormadinonacetats in der ersten Phase immer geringer als in der zweiten Phase, aber konstant ist, wobei die Menge EE pro Tages-Einheit über beide Phasen konstant bei 20 µg liegt.

Bei einem Dreiphasen-Kontrazeptivum beginnt der Einnahmezyklus vorzugsweise mit einer täglichen Einnahme einer Tages-Einheit enthaltend 1 bis 2 mg Chlormadinonacetat neben EE über einen Zeitraum von 6 bis 7 Tagen, gefolgt von einer täglichen Einnahme einer Tages-Einheit enthaltend 2 mg Chlormadinonacetat neben EE über einen Zeitraum von 5 bis 9 Tagen und endet mit einer täglichen Einnahme einer Tages-Einheit enthaltend 2 bis 3 mg Chlormadinonacetat über einen Zeitraum von 5 bis14 Tagen. Auch bei einem Dreiphasen-Kontrazeptivum enthalten die Tages-Einheiten von Phase zu Phase jeweils unterschiedliche Mengen an Chlormadinonacetat, wobei von der ersten bis zur dritten Phase die Menge an Chlormadinonacetat pro Tages-Einheit ansteigt, während die Menge innerhalb einer Phase konstant bleibt, ebenso wie die Menge EE über alle Phasen.

Sowohl bei einem Zweiphasen-Kontrazeptivum als auch bei einem Dreiphasen-Kontrazeptivum enthalten die hormonalen Tages-Einheiten vorzugsweise jeweils 20 µg Ethinylestradiol.

Weiterhin ist zur Erzielung einer maximalen Sicherheit in der kontrazeptiven Wirkung die Einhaltung einer aufeinanderfolgenden Einnahme von 21 bis 25 hormonhaltigen Tages-Einheiten pro Zyklus besonders wichtig.

In einem Einnahmezyklus kann vor oder nach, vorzugsweise nach einer Einnahmephase von 21 bis 25 hormonhaltigen Tages-Einheiten, eine tägliche Einnahme von 7 bis 3 hormonfreien Tages-Einheiten erfolgen.

Besonders bevorzugt ist ein erfindungsgemäßes monophasiges Kontrazeptivum.

Die Tages-Einheiten des erfindungsgemäßen Kontrazeptivums können vorzugsweise in der Form von Tabletten vorliegen. Herstellungsmethode für solche Tages-Einheiten sind dem Fachmann bekannt. Als Zusatzstoffen neben der Kombination aus Chlormadinonacetat und Ethinylestradiol können bekannte Hilfsstoffe verwendet werden. Vorzugsweise wird das erfindungsgemäße Kontrazeptivum für einen Einnahmezyklus in einem Blister, vorzugsweise mit Kennzeichnung der jeweils einzunehmenden Tages-Einheit, verpackt und vorzugsweise als eine Packung enthaltend mindestens einen solchen Blister, vorzugsweise mindestens 3 Blister für 3 Einnahmezyklen, marktgeführt.

### Beispiele

### Beispiel 1:

**Zusammensetzung**

| | Pro Tablette | Pro Charge |
|---|---|---|
| Ethinylestradiol | 0,020 mg | 0,002 kg |
| Chlormadinonacetat | 2,000 mg | 0,200 kg |
| Povidon K30 | 3,000 mg | 0,300 kg |
| Lactose | 31,980 mg | 3,198 kg |
| Maisstärke | 12,000 mg | 1,200 kg |
| Magnesiumstearat | 0,500 mg | 0,050 kg |
| Hochdisperses Siliciumdioxid | 0,500 mg | 0,050 kg |

Ethinylestradiol (EE) und Povidone K 30 (Polyvinylpyrrolidon) wurden in 600 ml Ethanol gelöst. Chlormadinonacetat (Partikelgröße 90% < 50 µm), Lactose und Maisstärke wurden in einem Mischer/Granulierer (Diosna P25) 5 min gemischt und anschließend mit der ethanolischen EE/PVP Lösung durchfeuchtet und gemischt. Die feuchte Masse wurde durch ein 3 mm Sieb getrieben und in einem Vakuumtrockenschrank getrocknet. Das trockene Granulat wurde durch ein 0,6 mm Sieb desagglomeriert, mit Magnesiumstearat und hochdispersem Siliciumdioxid gemischt und auf einer Tablettenpresse mit 5 mm Stempeln zu Tabletten mit einem Gewicht von 50 mg gepresst.

Die Tabletten wurden mit einem Lack auf Basis Methylhydroxypropylcellulose überzogen (z. B. Opadry YS-1-2184); Überzugsmasse 2 mg pro Tablette.

Von diesen Tabletten wurden 24 Stück als hormonhaltige Tages-Einheiten und entsprechend zusammengesetzte, aber hormonfreie Tabletten als hormonfreie Tages-Einheiten in einem Blister zu einem erfindungsgemäßen Kontrazeptivum verpackt.

### Beispiel 2:

**Zusammensetzung**

| | Pro Tablette | Pro Charge |
|---|---|---|
| Ethinylestradiol | 0,015 mg | 0,0015 kg |
| Chlormadinonacetat | 2,000 mg | 0,2000 kg |
| Povidon K30 | 3,000 mg | 0,3000 kg |
| Lactose | 32,985 mg | 3,2985 kg |
| Maisstärke | 12,000 mg | 1,2000 kg |
| Magnesiumstearat | 0,500 mg | 0,0500 kg |
| Hochdisperses Siliciumdioxid | 0,500 mg | 0,0500 kg |

Ethinylestradiol (EE) und Povidone K 30 (PVP) wurden in 600 ml Ethanol gelöst. Chlormadinonacetat (Partikelgröße 90% <50µm), Lactose und Maisstärke wurden in einem Mischer/Granulierer (Diosna P25) 5 min gemischt und anschließend mit der ethanolischen EE/PVP Lösung durchfeuchtet und gemischt. Die feuchte Masse wurde durch ein 3 mm Sieb getrieben und in einem Vakuumtrockenschrank getrocknet. Das trockene Granulat wurde durch ein 0,6 mm Sieb desagglomeriert, mit Magnesiumstearat und hochdispersem Siliciumdioxid gemischt und auf einer Tablettenpresse mit 5 mm Stempeln zu Tabletten mit einem Gewicht von 50 mg gepreßt.
Die Tabletten wurden mit einem Lack auf Basis Methylhydroxypropylcellulose mit folgender Zusammensetzung überzogen (Überzugsmasse 2 mg pro Tablette)

| | |
|---|---|
| Methylhydroxypropylcellulose 6 mPa · s, | 0,1351 kg |
| Polyethylenglykol 6000 | 0,0395 kg |
| Propylenglykol | 0,0054 kg |
| Gereinigtes Wasser | 1,6200 kg |

Wie in Beispiel 1 angegeben, wurden 24 Tabletten als hormonhaltige Tages-Einheiten und entsprechend zusammengesetzte 4 hormonfreie Tabletten in einem Blister zu einem erfindungsgemäßen Kontrazeptivum verpackt.

### Beispiel 3:

**Zusammensetzung**

| | Pro Tablette | Pro Charge |
|---|---|---|
| Ethinylestradiol | 0,015 mg | 0,0015 kg |
| Chlormadinonacetat | 3,000 mg | 0,3000 kg |
| Povidon K30 | 4,000 mg | 0,4000 kg |
| Lactose | 63,485mg | 6,3485 kg |
| Maisstärke | 10,000 mg | 1,0000 kg |
| Magnesiumstearat | 0,500 mg | 0,0500 kg |

Ethinylestradiol (EE) und Povidone K 30 (PVP) wurden in 950 ml Ethanol gelöst. Chlormadinonacetat (Partikelgröße 90% <50µm), Lactose und Maisstärke wurden in einem Mischer/Granulierer (Diosna P25) 5 min gemischt und anschließend mit der ethanolischen EE/PVP Lösung durchfeuchtet und gemischt. Die feuchte Masse wurde durch ein 3 mm Sieb getrieben und in einem Vakuumtrockenschrank getrocknet. Das trockene Granulat wurde durch ein 0,6 mm Sieb desagglomeriert, mit Magnesiumstearat gemischt und auf einer Tablettenpresse mit 6 mm Stempeln zu Tabletten mit einem Gewicht von 80 mg gepresst.
Die Tabletten wurden mit einem Lack auf Basis Methylhydroxypropylcellulose mit folgender Zusammensetzung überzogen (Überzugsmasse 2 mg pro Tablette)

| | |
|---|---|
| Methylhydroxypropylcellulose 6 mPa · s, | 0,1351 kg |
| Polyethylenglykol 6000 | 0,0395 kg |
| Propylenglykol | 0,0054 kg |
| Gereinigtes Wasser | 1,6200 kg |

Wie in Beispiel 1 angegeben, wurden 24 Tabletten als hormonhaltige Tages-Einheiten und entsprechend zusammengesetzte 4 hormonfreie Tabletten in einem Blister zu einem erfindungsgemäßen Kontrazeptivum verpackt.

### Beispiel 4:

### 2-Phasen Kontrazeptivum

a)

**Zusammensetzung der 1. Phase:**

| | Pro Tablette |
|---|---|
| Ethinylestradiol | 0,020 mg |
| Chlormadinonacetat | 2,000 mg |
| Povidon K30 | 3,000 mg |
| Lactose | 31,980 mg |
| Maisstärke | 12,000 mg |
| Magnesiumstearat | 0,500 mg |
| hochdisperses Siliciumdioxid | 0,500 mg |

Ethinylestradiol (EE) und Povidon K 30 (Polyvinylpyrrolidon) wurden in 600 ml Ethanol gelöst. Chlormadinonacetat (Partikelgröße 90% < 50 µm), Lactose und Maisstärke wurden in einem Mischer/Granulierer (Diosna P25) 5 min gemischt und anschließend mit der ethanolischen EE/PVP Lösung durchfeuchtet und gemischt. Die feuchte Masse wurde durch ein 3 mm Sieb getrieben und in einem Vakuumtrockenschrank getrocknet. Das trockene Granulat wurde durch ein 0,6 mm Sieb desagglomeriert, mit Magnesiumstearat und hochdispersem Siliciumdioxid gemischt und auf einer Tablettenpresse mit 5 mm Stempeln zu Tabletten mit einem Gewicht von 50 mg gepresst.
b)

**Zusammensetzung der 2. Phase:**

| | Pro Tablette |
|---|---|
| Ethinylestradiol | 0,020 mg |
| Chlormadinonacetat | 3,000 mg |
| Povidon K30 | 3,000 mg |
| Lactose | 30,980 mg |
| Maisstärke | 12,000 mg |
| Magnesiumstearat | 0,500 mg |
| hochdisperses Siliciumdioxid | 0,500 mg |

Die 50 mg Tabletten wurden, wie unter a) angegeben hergestellt.

Die nach a) bzw. b) hergestellten Tabletten wurden mit einem Lack auf Basis Methylhydroxypropylcellulose überzogen (z. B. Opadry YS-1-2184 vom Hersteller Colorcon); Überzugsmasse 2 mg.

Für ein erfindungsgemäßes Kontrazeptivum wurden 12 nach a) hergestellte Tabletten zur Einnahme zu Beginn des Einnahmezyklus, 12 weitere nach b) hergestellte Tabletten und 4 hormonfreie, aber sonst entsprechend zusammengesetzte Tabletten in einem zur Einnahme markierten Blister verpackt.

## Patentansprüche

1. Hormonales Kontrazeptivum aus 21 bis 25 hormonhaltigen Tages-Einheiten zur Verabreichung einer Kombination aus einem Östrogen und einem Gestagen pro Tageseinheit und 7 bis 3 hormonfreie Tages-Einheiten an Frauen, **dadurch gekennzeichnet, dass** die hormonhaltigen Tages-Einheiten eine Kombination aus Ethinylestradiol in einer Menge von 10 bis 20 µg und Chlormadinonacetat in einer Menge von 1 bis 4 mg enthalten.

2. Hormonales Kontrazeptivum gemäß Anspruch 1, zur Verabreichung an Frauen über 35 Jahre.

3. Hormonales Kontrazeptivum gemäß Anspruch 1 oder 2, **dadurch gekennzeichnet, dass** die hormonhaltigen und die hormonfreien Tages-Einheiten die Form von Tabletten aufweisen.

4. Hormonales Kontrazeptivum gemäß einem der Ansprüche 1 bis 3, **dadurch gekennzeichnet, dass** die hormonhaltigen Tages-Einheiten als Kombination 10 bis 20 µg Ethinylestradiol und 1 bis 3 mg Chlormadinonacetat enthalten, wobei jede der hormonhaltigen Tages-Einheiten jeweils dieselbe Menge an Ethinylestradiol bzw. an Chlormadinonacetat enthält.

5. Hormonales Kontrazeptivum gemäß Anspruch 4, **dadurch gekennzeichnet, dass** die hormonhaltigen Tages-Einheiten als Kombination jeweils 20 µg Ethinylestradiol und jeweils 2 mg Chlormadinonacetat enthalten.

6. Hormonales Kontrazeptivum gemäß Anspruch 4, **dadurch gekennzeichnet, dass** die hormonhaltigen Tages-Einheiten als Kombination jeweils 20 µg Ethinylestradiol und jeweils 3 mg Chlormadinonacetat enthalten.

7. Hormonales Kontrazeptivum gemäß Anspruch 4, **dadurch gekennzeichnet, dass** die hormonhaltigen Tages-Einheiten als Kombination jeweils 15 µg Ethinylestradiol und jeweils 2 mg Chlormadinonacetat enthalten.

8. Hormonales Kontrazeptivum gemäß einem der Ansprüche 1 bis 7 aus 21 bis 25 hormonhaltigen Tages-Einheiten zur aufeinanderfolgenden Verabreichung und aus 7 bis 3 hormonfreien Tages-Einheiten zur darauffolgenden, aufeinanderfolgenden Verabreichung.

9. Hormonales Kontrazeptivum gemäß einem der Ansprüche 1 bis 7 aus 21 bis 24 hormonhaltigen Tages-Einheiten zur aufeinanderfolgenden Verabreichung und aus 7 bis 4 hormonfreien Tages-Einheiten zur darauffolgenden, aufeinanderfolgenden Verabreichung.

## Claims

1. Hormonal contraceptive comprising 21 to 25 hormone-containing daily units of a combination containing an estrogen component and a progestogen component, and 7 to 3 hormone-free daily units for administration to women, wherein the hormone-containing daily units comprise a combination containing ethinyloestradiol in an amount of 10 to 20 µg and chlormadinone acetate in an amount of 1 to 4 mg.

2. Hormonal contraceptive according to claim 1 for administration to women above age 35.

3. Hormonal contraceptive according to claim 1 or claim 2, **characterized in that** hormone containing- and hormone-free daily units take the form of tablet.

4. Hormonal contraceptive according to any of claims from 1 to 3, **characterized in that** the hormone-containing daily units as a combination contain ethinyloestradiol in an amount of 10 to 20 µg and chlormadidone acetate in an amount of 1 to 3 mg, wherein each of the hormone-containing daily units contain the same amount of ethinyloestradiol or of chlormadidone acetate.

5. Hormonal contraceptive according to claim 4, **characterized in that** the hormone-containing daily units each contain 20 µg of ethhinyloestradiol and 2 mg of chlormadidone acetate.

6. Hormonal contraceptive according to claim 4, **characterized in that** the hormone-containing daily units each contain 20 µg of ethhinyloestradiol and 3 mg of chlormadidone acetate.

7. Hormonal contraceptive according to claim 4, **characterized in that** the hormone-containing daily units each contain 15 µg of ethhinyloestradiol and 2 mg of chlormadidone acetate.

8. Hormonal contraceptive according to any of claims from 1 to 7 for consecutive administration of hormone-containing daily units on 21 to 25 days, followed by an interval of consecutive administration of hormone-free daily units on 7 to 3 days.

9. Hormonal contraceptive according to any of claims 1-7 for consecutive administration of hormone-containing daily units on 21 to 24 days, followed by an interval of consecutive administration of hormone-free daily units on 7 to 4 days.

## Revendications

1. Contraceptif hormonal constitué par 21 à 25 unités journalières contenant des hormones pour l'administration d'une combinaison d'un oestrogène et d'un gestagène par unité journalière et de 7 à 3 unités journalières exemptes d'hormones à des femmes, **caractérisé en ce que** les unités journalières contenant des hormones contiennent une combinaison d'éthinylestradiol en une quantité de 10 à 20 µg et d'acétate de chlormadinone en une quantité de 1 à 4mg.

2. Contraceptif hormonal selon la revendication 1 pour l'administration à des femmes au-dessus de 35 ans.

3. Contraceptif hormonal selon la revendication 1 ou 2, **caractérisé en ce que** les unités journalières contenant des hormones et celles exemptes d'hormones présentent la forme de comprimés.

4. Contraceptif hormonal selon l'une quelconque des revendications 1 à 3, **caractérisé en ce que** les unités journalières contenant des hormones contiennent comme combinaison 10 à 20 µg d'éthinylestradiol et 1 à 3 mg d'acétate de chlormadinone, chacune des unités journalières contenant des hormones contenant la même quantité d'éthinylestradiol ou d'acétate de chlormadinone.

5. Contraceptif hormonal selon la revendication 4, **caractérisé en ce que** les unités journalières contenant des hormones contiennent comme combinaison à chaque fois 20 µg d'éthinylestradiol et à chaque fois 2 mg d'acétate de chlormadinone.

6. Contraceptif hormonal selon la revendication 4, **caractérisé en ce que** les unités journalières contenant des hormones contiennent comme combinaison à chaque fois 20 µg d'éthinylestradiol et à chaque fois 3 mg d'acétate de chlormadinone.

7. Contraceptif hormonal selon la revendication 4, **caractérisé en ce que** les unités journalières contenant des hormones contiennent comme combinaison à chaque fois 15 µg d'éthinylestradiol et à chaque fois 2 mg d'acétate de chlormadinone.

8. Contraceptif hormonal selon l'une quelconque des revendications 1 à 7, constitué par 21 à 25 unités journalières contenant des hormones pour l'administration successive et par 7 à 3 unités journalières exemptes d'hormones pour une administration consécutive, successive.

9. Contraceptif hormonal selon l'une quelconque des revendications 1 à 7, constitué par 21 à 24 unités journalières contenant des hormones pour l'administration successive et par 7 à 4 unités journalières exemptes d'hormones pour une administration consécutive, successive.
